# EUROPEAN PATENT APPLICATION

(11) **EP 3 181 683 A1**
(43) Date of publication of application: **21.06.2017**
(21) Application number: 15200520.3
(22) Date of filing: 16.12.2015
(51) Int. Cl.: C12N 1/04, C12N 1/20, C12N 1/38

(54) **A METHOD OF ENHANCING THE SURVIVAL AND RECOVERY OF CERTAIN STRAINS OF BACTERIA**

(71) Applicant: University College Cork - National University of Ireland, Cork, Cork (IE)
(72) Inventor: Van Sinderen, Douwe, Co. Cork (IE); O'Connell Motherway, Mary, Co. Cork (IE); O'Callaghan, Amy, Co. Cork (IE)
(74) Representative: Purdy, Hugh Barry

(57) **Abstract**

A method of growing an acid or oxygen sensitive strain belonging to the genus *Bifidobacterium* or *Lactobacillus,* comprising the step of incubating the oxygen sensitive strain of *Bifidobacterium* or *Lactobacillus* in a fermentation medium comprising a phenolic acid donating natural hemicellulose component as a fermentable energy source under growth favouring conditions and a for a period of time sufficient to increase the bacterial cell mass, and recovering the grown *Bifidobacterium* or *Lactobacillus,* wherein the oxygen-sensitive strain of *Bifidobacterium* or *Lactobacillus* exhibits enhanced survival post-fermentation compared with the same bacteria grown in a conventional fermentation medium.

## Description

### Technical Field

The invention relates to methods of growing, recovering and storing acid or oxygen sensitive bacteria, and fermentation media for acid or oxygen sensitive bacteria.

### Background to the Invention

In order to render their beneficial effects, it is believed that probiotic strains of bacteria need to maintain their viability during production, storage, and passage through the human gastrointestinal tract. For probiotics to be therapeutically effective, it has been suggested that such a product should contain at least 6log cfu/g of viable probiotic bacteria until the end of its shelf life. However, a number of factors compromise the viability of probiotics within food and supplementation products besides those faced during gastrointestinal passage. Potentially beneficial but relatively susceptible strains are prone to loss of viability during the harsh conditions faced during production and subsequent processing, as well as during storage in fermented food products. For example, various strains of *Bifidobacterium* or *Lactobacillus* are routinely incorporated into dairy food products like yoghurt, where the strains face several challenges to their survival which limits their potential health benefits. The major reason for poor survival under these conditions is toxicity due to oxygen and reactive oxygen species (ROS), as well as acid-induced toxicity due to the presence of organic acids that reduce the pH of the product. These probiotic strains are mostly microaerophiles or strict anaerobes of intestinal origin, thus making them highly susceptible to loss of viability when exposed to organic acids/low pH, oxygen and/or ROS in food products.

It is an object of the invention to overcome at least one of the above-referenced problems.

### Statements of Invention

Broadly, the Applicant has discovered that growing or storing acid or oxygen sensitive bacteria such as an anaerobic or microaerophilic gut commensals in the presence of certain plant fibres enhances the viability and survival of the strains. In particular, the Applicant has discovered that production of certain acid/oxygen-sensitive bifidobacteria in a fermentation process using certain plant fibres as the sole carbon/energy source produces bacteria that exhibit enhanced survival post-fermentation. The plant fibres that cause this effect are fermentable fibres that contain a reservoir of phenolic acids (such as ferulic acid) - an example of such fibres is arabinoxylan, a plant fibre found in the cell walls of many plants, including cereals. Without being bound by theory, it is believed that ferulic acid of arabinoxylan can be used as an electron sink by certain bifidobacteria or as a reducing agent of reactive oxygen species ROS, events that provide a protective effect that inhibits/prevents direct damage to the bacteria by oxygen ROS or organic acids, and/or causing a sudden drop in pH which would be detrimental to growth conditions and survival. This effect is not observed when bacteria are grown in the presence of a carbohydrate that lacks ferulic acid. The invention therefore relates to methods of growing acid/oxygen-sensitive bacteria, methods of drying acid/oxygen-sensitive bacteria, methods of reconstitution of dried acid/oxygen-sensitive bacteria, and general methods of storing bacteria, all of which involve bringing the bacteria into contact with the plant fibres having a reservoir of phenolic acids. In another, linked, aspect, the Applicant has discovered that the recovery of stressed bacteria can be enhanced by the presence of ferulic acid. Examples of stressed bacteria are oxygen-sensitive bacteria located in or exposed to oxygen-containing environments such as faeces or sewage, or bacteria stressed as a result of a physical treatment such as electroporation. Thus, the invention also relates to methods of promoting the recovery of stressed bacteria comprising the step of incubating or recovering the bacteria in a medium containing ferulic acid.

In one aspect, the invention relates to a method of enhancing the survival or recovery of an acid or oxygen sensitive bacterial strain comprising a step of bringing the bacteria into contact with a phenolic acid-donating natural hemicellulose component or a phenolic acid.

In one embodiment, the bacteria are brought into contact with the phenolic acid-donating natural hemicellulose component in a fermentation medium.

In one embodiment, the bacteria are brought into contact with the phenolic acid-donating natural hemicellulose component in a storage medium.

In one embodiment, the bacteria are brought into contact with the phenolic acid-donating natural hemicellulose component in a reconstitution medium, where the bacteria are in a dried format prior to reconstitution.

In one embodiment, the bacteria are brought into contact with the phenolic acid-donating natural hemicellulose component in a microcapsule.

In another aspect, the invention provides a method of growing an acid or oxygen sensitive strain of bacteria, comprising the step of incubating the acid or oxygen sensitive strain of bacteria in a fermentation medium comprising a phenolic acid-donating natural hemicellulose component as a fermentable energy source under growth favouring conditions and a for a period of time sufficient to increase the cell mass of the bacteria, and recovering the bacteria.

In one embodiment, the bacteria are subsequently dried.

In one embodiment, the dried bacteria are stored in a storage medium, wherein the storage medium comprises the phenolic acid-donating natural hemicellulose component.

In one embodiment, the bacteria are dried in the presence of a phenolic acid-donating natural hemicellulose component. For example, the bacteria may be mixed with the phenolic acid-donating natural hemicellulose component prior to drying.

In one embodiment, the dried bacteria are reconstituted in a reconstitution medium comprising the phenolic acid-donating natural hemicellulose component.

In another aspect, the invention provides a method of storing an acid or oxygen sensitive strain of bacteria, comprising the step of storing the acid or oxygen sensitive strain of bacteria in a storage medium comprising a phenolic acid-donating natural hemicellulose component.

In another aspect, the invention provides a method of drying an acid or oxygen sensitive strain of bacteria, comprising the step of drying the acid or oxygen sensitive strain of bacteria in a drying medium comprising a phenolic acid-donating natural hemicellulose component.

In one embodiment, the dried acid or oxygen sensitive strain of bacteria in a reconstitution medium comprising a phenolic acid-donating natural hemicellulose component.

In another aspect, the invention provides a method of reconstitution of a dried acid or oxygen sensitive strain of bacteria, comprising the step of reconstitution of the dried acid or oxygen sensitive strain of bacteria in a reconstitution medium comprising a phenolic acid-donating natural hemicellulose component.

In one embodiment, the bacterium is a probiotic micro organism. In one embodiment, the bacterium is selected from an oxygen-sensitive *Bifidobacterium* or an oxygen-sensitive *Lactobacillus*.

In one embodiment, the bacterium is selected from an acid or oxygen-sensitive probiotic *Bifidobacterium* or an acid or oxygen-sensitive probiotic *Lactobacillus.*

In one embodiment, the phenolic acid-donating natural hemicellulose component comprises a reservoir of ferulic acid or another hydroxycinnamic acid.

In one embodiment, the phenolic acid-donating natural hemicellulose component is an arabinoxylan.

In one embodiment, the arabinoxylan is derived from a cereal grain.

In one embodiment, the arabinoxylan has a purity of at least 70%. In one embodiment, the arabinoxylan has a purity of at least 80%. In one embodiment, the arabinoxylan has a purity of at least 90%. Purity as applied to arabinoxylan means that amount of impurities in an arabinoxylan product, thus a 90% purity means that 90% by weight of the arabinoxylan is in fact arabinoxylan, with the remaining 10% representing impurities such as other plant fibres.

In one embodiment, the phenolic acid-donating natural hemicellulose component comprises at least 50% of the fermentable energy source in the fermentation medium.

In one embodiment, the phenolic acid-donating natural hemicellulose component comprises at least 75% of the fermentable energy source in the fermentation medium.

In one embodiment, the grown bacterial strains belonging to *Bifidobacterium* and/or *Lactobacillus* are dried.

In one embodiment, the grown, stored, dried, or reconstituted bacterial strains belonging to *Bifidobacterium* and/or *Lactobacillus* are incorporated into a comestible product which additionally comprises the phenolic acid-donating natural hemicellulose.

In one embodiment, the comestible product is a food product, beverage, food supplement, or an oral drug.

The invention also provides a culture of bacteria produced according to a method of the invention.

The invention also provides a culture of bacteria in a dried format produced according to a method of the invention.

The invention also provides a culture of bacteria in a dried and reconstituted format produced according to a method of the invention.

In another aspect, the invention provides a fermentation medium suitable for growing bacteria comprising a fermentable energy source, and optionally one or more growth factors selected from free amino acid, peptides, nucleotides, vitamins, and/or minerals, and characterised in that the fermentable energy source comprises a phenolic acid-donating natural hemicellulose component.

In one embodiment, the phenolic acid-donating natural hemicellulose comprises at least 50% of the fermentable energy source in the fermentation medium.

In one embodiment, the phenolic acid-donating natural hemicellulose component comprises at least 75% of the energy source in the fermentation medium.

In one embodiment, the fermentation medium comprises free amino acids, peptides, nucleotides, and optionally a vitamin and/or a mineral. In one embodiment, the fermentation medium consists essentially of a phenolic acid-donating natural hemicellulose component

In another aspect, the invention provides a microcapsule comprising an acid or oxygen sensitive strain of bacteria and a phenolic acid-donating natural hemicellulose component. In one embodiment, the microcapsule has a core-shell morphology, in which the core comprises the acid or oxygen sensitive strain of bacteria and optionally the phenolic acid-donating natural hemicellulose component. In one embodiment, the shell comprises the phenolic acid-donating natural hemicellulose component. In one embodiment, the microcapsule comprises a continuous matrix and the acid or oxygen sensitive strain of bacteria and optionally the phenolic acid-donating natural hemicellulose component dispersed throughout the matrix. In one embodiment, the matrix comprises phenolic acid-donating natural hemicellulose component.

The Applicant has also discovered that the recovery of acid or oxygen-sensitive bacteria that have been stressed by exposure to an oxygen-rich environment (e.g. faecal samples) or by physical stress (e.g. electroporation, plasmid transformation) can be promoted or improved by exposing the bacteria to a phenolic acid, especially a hydroxycinnamic acid such as ferulic acid. For example, environmental samples containing acid or oxygen sensitive bacteria may be stored in the presence of a phenolic acid, or bacteria may be isolated from the environmental sample in the presence of phenolic acid. This is relevant to many probiotic bacteria that are isolated from oxygen-exposed samples such as faeces or sewage plants, or exposed to stressful treatments such as electroporation.

Thus, in a further aspect, the invention relates to a method of improving the recovery of an acid or oxygen sensitive strain of bacteria that has been stressed comprising a step of incorporating a phenolic acid, for example a hydroxycinnamic acid, in the bacterial recovery/growth medium. In one embodiment, the bacteria is contained within a sample, for example an oxygen-exposed environmental sample such as faeces or sewage. In one embodiment, the bacteria has been subjected to a physical stress, such as electroporation. In one embodiment, the bacteria has been subjected to a chemical stress, such as being a low pH environment. In one embodiment, the sample containing the bacteria is stored (or transported) in a storage medium comprising a phenolic acid. In one embodiment, the storage medium comprises 0.1-1.0% (v/v) phenolic acid. In one embodiment, the bacteria is isolated from the sample in or on a bacterial growth medium comprising a phenolic acid. In one embodiment, the bacterial growth medium comprises 0.1-1.0% (v/v) phenolic acid.

In another aspect, the invention relates to a bacterial growth medium comprising a phenolic acid, optionally in an amount of 0.1-10% (v/v).

In one embodiment, the bacterial growth medium comprises 0.1 to 10% phenolic acid (v/v). In one embodiment, the bacterial growth medium comprises 0.1 to 1% phenolic acid (v/v). In one embodiment, the bacterial growth medium comprises 0.1 to 0.5% phenolic acid (v/v).

In one embodiment, the bacterial growth medium is a solid, liquid or gel. In one embodiment, the growth medium is a nutrient broth. In one embodiment, the growth medium is an agar plate.

### Definitions:

"Acid or oxygen sensitive strain" as applied to a cell for example a probiotic bacteria or a Bifidobacterium means a strain of bacteria that is sensitive to acid, oxygen or reactive oxygen species (ROS). Examples include anaerobic bacteria and microaerophiles. "Oxygen-sensitive" means that the bacteria will not survive exposure to atmospheric levels of oxygen.

"Bacteria" means any bacteria and encompasses bacteria in the plural or singular (bacterium). In one embodiment, the bacteria is a non-pathogenic bacteria. In one embodiment, the bacterium is a pathogenic bacteria. In one embodiment, the bacterium is an anaerobe or a microaerophile. In one embodiment, the bacterium is selected from the genera *Lactobacillus, Bifidobacterium, Pediococcus*, *Propionibacterium*, *Enterococcus*, *Bacillus*, and *Streptococcus.* In one embodiment, the bacterium is a probiotic bacteria. In one embodiment, the bacterium is derived from the mammalian or human gut. In one embodiment, the bacterium is a *Bifidobacterium.* In one embodiment, the bacterium is a *Bifidobacterium longum.* In one embodiment, the bacterium is a *Bifidobacterium longum* subspecies *longum.*

"Probiotic bacteria" means a strain of bacteria that is non-pathogenic, bile tolerant, and which when administered in a viable form to a mammalian host confers a health benefit on the host. Probiotic strains are generally of the genera *Lactobacillus* and *Bifidobacterium,* and to a lesser extent *Pediococcus*, *Propionibacterium, Enterococcus*, *Bacillus*, *Streptococcus* and *Saccharomyces.* Specific examples of probiotic bacteria include: *Bifidobacterium animalis* subsp. *lactis* Bf-6; *Lactobacillus johnsonii* La-1; *Bifidobacterium bifidum* BF2; *Bifidobacterium longum* BG3; *Bifidobacterium lactis* BB12; *Bifidobacterium animalis* subsp. *lactis* B-420; *Bifidobacterium lactis* HN019; *Pediococcus pentosaceus* PP; *Lactobacillus rhamnosus* LR3; *Bifidobacterium breve* M16V; *Bifidobacterium pseudolongum* M-602; and *Lactobacillus rhamnosus* HN001.

"Phenolic acid donating natural hemicellulose component" means a natural plant fibre material that can be fermented by bacteria and comprises a covalently bound phenolic acid such as ferulic acid or coumaric acid. Examples of phenolic acid-donating natural hemicellulose components include arabinoxylan, including arabinoxylo-oligosaccharides, arabinogalactan, and arabinan. This type of plant fibre is found in the cell wall of many plant cells and also found in other plant material such as plant seeds.

"Phenolic acid" refers to naturally occurring aromatic acids, in particular hydroxycinnamic acids, that are found in plant cells covalently attached to plant fibres such as hemicelluloses and lignocelluloses. Specific examples of phenolic acids include: ferulic acid, diferulic acid, caffeic acid and coumaric acid.

"Arabinoxylan" means a plant fibre found in the primary and secondary cell walls of plants including woods and cereal grains comprising a copolymer of xylose and arabinose and containing a reservoir of covalently bound phenolic acids, especially ferulic acid. In one embodiment, the arabinoxylan is derived from a cereal grain, for example wheat, maize, or rye. Examples of arabinoxylan and arabinoxylo-oligosaccharides are described in WO2011157968.

"Fermentation medium" means a composition of components designed to support the growth of a cell, be it a mammalian or bacterial cell. Generally, the medium will contain one or more energy sources, and optionally additional nutrients including one or more of free amino acids, vitamins, dairy protein, yeast extracts, minerals, and nucleotides. In one embodiment, the fermentation medium comprises a source of energy, free amino acid, and vitamins. In one embodiment, the fermentation medium is a liquid. In one embodiment, the fermentation medium has a low oxidation/reduction potential. In one embodiment, the fermentation medium has a pH value of from 5 to 8. In one embodiment, the fermentation medium has a pH value of from 6-7. In one embodiment, the fermentation medium is formulated for use in a batch or fed-batch reactor. In one embodiment, the fermentation medium comprises 0.1% to 10% phenolic acid donating natural hemicellulose. In one embodiment, the fermentation medium comprises 0.1% to 1% phenolic acid donating natural hemicellulose. In one embodiment, the fermentation medium comprises 0.1% to 0.5% phenolic acid donating natural hemicellulose. In one embodiment, the fermentation medium comprises 0.25% to 0.5% phenolic acid donating natural hemicellulose.

"Fermentable energy source" means a sugar that that bacteria can grow on. Examples include glucose, fructose, mannose, plant fibres, and other sugars. In one embodiment, the fermentable energy source is predominantly the phenolic acid donating natural hemicellulose. In one embodiment, the phenolic acid donating natural hemicellulose is the sole fermentable energy source in the fermentation medium.

"Growth favouring conditions" means conditions conducive to growth of bacteria. The specific parameters vary between different bacteria. Generally the conditions include elevated temperature, and a pH that is neither too acidic nor too alkaline, typically in the 5-8 range. Generally, the conditions are maintained until a desired cell mass of bacteria is obtained, at which point one or more of the conditions are changed to prevent further growth.

"Dried" as applied to bacteria means that the bacteria are subjected to a dehydration process. Specific methods of drying cell mass include freeze-drying (lyophilisation), spray drying, fluidized bed drying. Other methods will be apparent to a person skilled in the art. Generally, as a result of the drying process, the water context of the bacteria is reduced to less than 5% (v/w). Commercial strains of bacteria are often stored in dried format, and then reconstituted in a reconstitution medium prior to use, for example, use in a food product.

"Reconstitution medium" means a liquid in which dried bacteria are re-hydrated prior to use or further storage. Typically, the reconstitution medium comprises water optionally in combination with growth factors or a carbon/energy source for the bacteria.

"Comestible product" means a man-made product that is intended to be eaten by mammals, especially humans. Examples include foods, beverages, food supplements and oral drugs.

"Fermented food product" means a food product that contains lactic acid producing bacteria. Examples include many dairy products such as yoghurts, cheeses.

"Microcapsule" means a micron-sized solid particle having a maximum dimension of less than 1000 microns and which contains an oxygen sensitive strain of bacteria along with a phenolic acid donating natural hemicellulose. The microcapsule may be a microparticle having a continuous matrix and bacteria dispersed throughout the matrix. The hemicellulose may also be dispersed throughout the matrix along with the bacteria, or may form part of the matrix. The microcapsule may have a core-shell morphology, in which the bacteria are disposed within the core, and the hemicellulose is also disposed within the core or is disposed within the shell matrix. The matrix may be formed of or comprise the hemicellulose, or another matrix forming material such as whey, casein, starch, alginate, gum, and vegetable protein. Methods for forming microcapsules will be known to a person skilled in the art, and can be prepared from a single feed stream which is formed into droplets (by spraying, atomising, or passing through an encapsulator) and hardened in hot air (spray drying) or in a gelation bath (cold gelation). Specific methods for producing microcapsules are described in WO2008017962, WO2014198787, WO2010111347, and CN101816418.

"Oxygen rich sample" means a material that servers as a reservoir of bacteria, especially probiotic bacteria, that is rich in oxygen or reactive oxygen species. Examples include sludge from sewage treatment plants and faeces.

"Bacterial growth medium" refers to media commonly used for the growth of bacteria, such as agar plates and nutrient broths and comprising an energy/carbon source for the bacteria along with other components conducive to bacterial growth such as amino acids, vitamins, minerals, yeast extract and certain salts. The medium may be solid, liquid or gel.

### Brief Description of the Figures

**Figure 1****.** Preferential carbohydrate utilisation of *B. longum* subsp. *longum* strains NCIMB8809, CCUG30698 or JCM1217 during growth in MRS supplemented with 0.05% cysteine HCL and 0.5 % w/v of a particular carbohydrate source.
**Figure 2****.** Survival of post fermentation cultures of *B. longum* subsp. *longum* strains NCIMB8809, CCUG30698, JCM1217 during storage at 37°C or 4°C.
**Figure 3.** Recovery of Bifidobacteria from 2 infant faecal samples by plating on TOS propionate agar supplemented with 100 µg ml⁻¹ mupirocin and 50µg ml⁻¹ nystatin (TOS-MN) and TOS- MN supplemented with 2 mM Ferulic acid. The numbers (cfu g⁻¹ feces) recovered from each sample on TOS-MN is indicated by the blue bars while the increase in cell numbers recovered on TOS- MN supplemented with 2 mM Ferulic acid is indicated by the additional orange bar.

### Detailed Description of the Invention

### Materials and methods

### Preferential carbohydrate utilisation, and survival of B. longum subsp. longum during storage at 37°C or 4°C

*Bacterial strains and culture conditions.* The bacterial strains used in this study are *B. longum* subsp. *longum* NCIMB8809, *B. longum* subsp. *longum* CCUG30698, *B. longum* subsp. *longum* JCM1217. Bifidobacterial strains were routinely cultured in reinforced clostridial medium (RCM; Oxoid Ltd, Basingstoke, Hampshire, United Kingdom). Carbohydrate utilization by bifidobacteria was examined in de Man Rogosa and Sharpe Medium (MRS) prepared from first principles (de Mann, Rogosa and Sharpe, 1960). Prior to inoculation MRS was supplemented with cysteine-HCl (0.05 % w/v). The carbohydrates used were rye arabinoxylan (AX-R), wheat arabinoxylan (AX-W), arabinan, debranched-arabinan, larchwood arabinogalactan, xylan, (purchased from Megazyme, Wicklow, Ireland,), xylo-oligosaccharides (obtained from Shandong Longlive Bio-Technology Co., Shandong, China), lactose, arabinose or xylose (purchased from Sigma). Bifidobacterial cultures were incubated at 37°C under anaerobic conditions which were maintained using an anaerobic hood (Davidson and Hardy, Belfast, Ireland).

*Analysis of carbohydrate utilisation by B. longum*. Filter-sterilised lactose, arabinose, xylose or xylo-oligosaccharide solutions were added to MRS medium to obtain a final sugar concentrations of 0.5%. AX-R, AX-W, arabinan, debranched arabinan, xylan or larchwood arabinogalactan were added directly to MRS prior to autoclaving to achieve final sugar concentrations of 0.5 %. Growth of *B. longum* strains in MRS supplemented with a particular carbohydrate and 0.05 % (wt/vol) cysteine-HCl was monitored at two or three hour intervals for 12 hours followed by a final time point at 24 hours by measuring the optical density at 600 nm (OD₆₀₀).

*Survival of B. longum during storage at 37°C or 4 °C* Following growth of *B. longum* for 24 hours, the number of viable cells was enumerated by performing serial dilutions and spread plating on reinforced clostridial agar (RCA, Oxoid, Basingstoke UK). Agar plates were incubated under anaerobic conditions at 37°C for 48 hours after which cell numbers were determined by plate counting. To evaluate culture viability at 37°C or 4°C, each culture was divided, half incubated anaerobically at 37°C while the second half was stored anaerobically at 4°C. At 48 h- 72 h intervals 150 ml aliquots were removed from each culture, serially diluted and the number of viable cells enumerated by plating on RCA as described above.

### Recovery and enumeration of Bifidobacteria from fecal samples.

*Isolation and enumeration of Bifidobacteria from fecal samples.* One gram of fecal material was defrosted on ice and suspended in 10 ml of phosphate buffered saline solution supplemented with 0.05 % v/v cysteine HCL (PBS-cys). Each sample was homogenised under anaerobic conditions, serially diluted in PBS-cys and viable bifidobacterial cell numbers enumerated by plating on TOS propionate agar supplemented with 100 µg ml⁻¹ mupirocin and 50 µg ml⁻¹ nystatin, or TOS propionate agar supplemented with 100 µg ml⁻¹ mupirocin, 50 µg ml⁻¹ nystatin and 2 mM Ferulic acid. Agar plates were incubated under anaerobic conditions at 37°C for 48 hours after which the number of viable bifidobacteria recovered under each condition was enumerated by plate counting. A representative number of colonies were selected from each agar medium and confirmed to be bifidobacteria by microscopic examination.

### Results

### Preferential carbohydrate utilisation, and survival of B. longum subsp. longum during storage at 37°C or 4°C

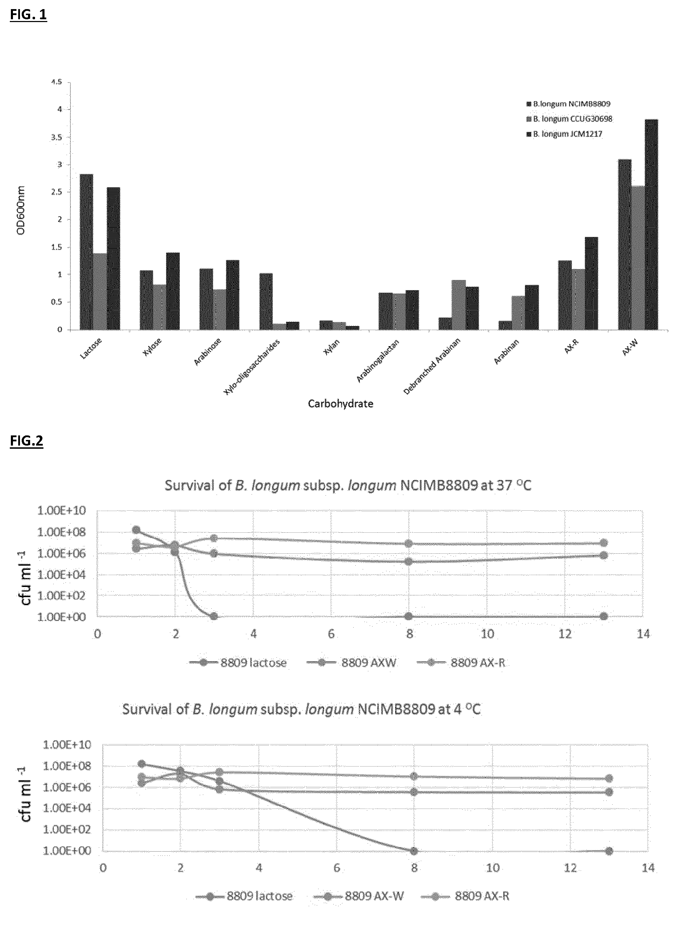

In order to establish if different *B. longum* strains have the ability to utilise arabinose- and/or xylose-containing carbohydrates as a sole carbon source, growth analysis was performed for three *B. longum* strains on a range of sugars. All three strains were capable of good growth in MRS supplemented with the monosaccharides arabinose or xylose (NB. lactose was used as the positive control). Vigorous growth of all three strains was also observed (final OD600 > 1.0) in MRS supplemented with AX-R or AX-W. *B. longum* strains CCUG30698 and JCM1217 displayed good growth (final OD600 > 0.6) in MRS supplemented with debranched arabinan or arabinan as the sole carbon/energy source, however, *B. longum* subsp. *longum* NCIMB 8809 displayed poor growth (final OD600 <0.2) in medium supplemented with these carbohydrates. Conversly, *B. longum* subsp. *longum* NCIMB 8809 displayed good growth (final OD600 >1.0) in medium supplemented xylo-oligosaccharides while *B. longum* strains CCUG30698 and JCM1217 exhibited no growth (final OD600 < 0.1) on this carbohydrate source. Each *B. longum* strain grew well in medium supplemented with Arabinogalactan reaching OD 600nm>0.6, however, medium supplemented with xylan did not support the growth of any of the three *B. longum* strains (Fig. 1). The observation that the bifidobacterial strains are incapable of utilising xylan, yet capable of growth on AX, suggests that the *B. longum* strains cleave and metabolise the arabinose substitutions from the latter polysaccharide. These results also suggest that there is a high level of inter-strain diversity among *B. longum* strains in terms of the metabolism of these carbohydrates.

### Survival of B. longum during storage at 37 °C or 4 °C

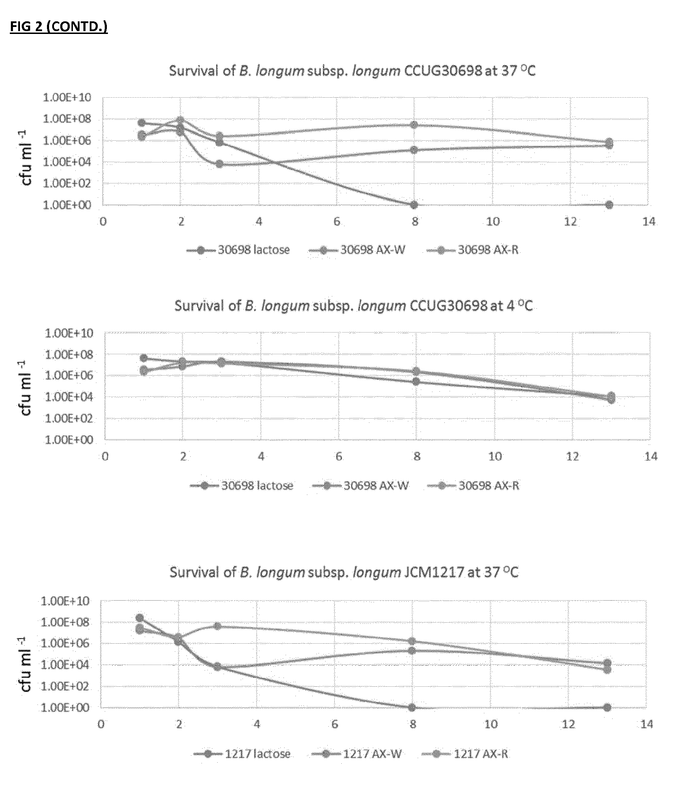

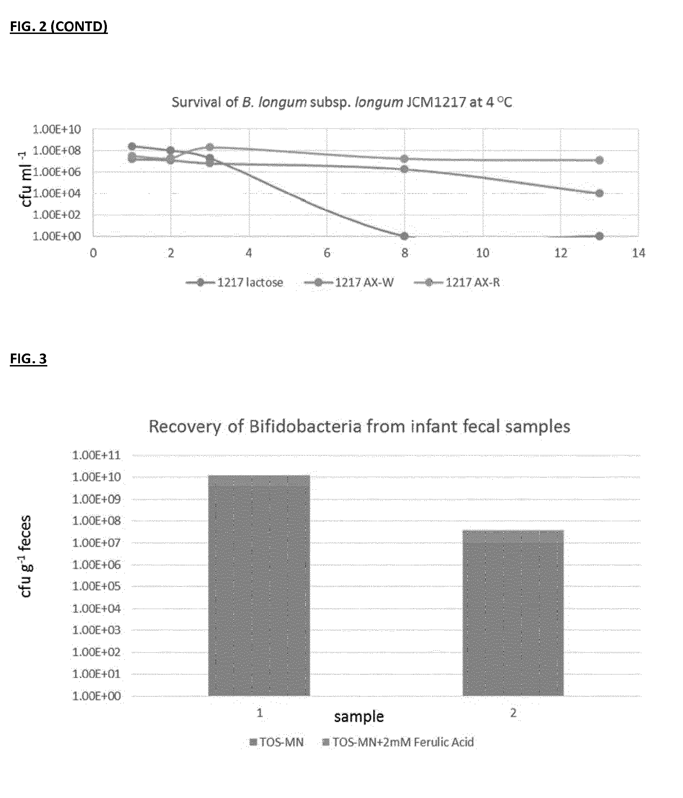

To establish if growth of *B. longum* strains in medium supplemented with AX-R or AX-W would enhance survival at during storage at 37°C or 4°C survival assays were performed. Post fermentation *B. longum* cultures (at 24h as described above) following growth in MRS supplemented with lactose, AX-R or AX-W were enumerated to determine the number of viable bifidobacterial cells. Subsequently each culture was divided, each half was stored at 37 °C or 4 °C under anaerobic conditions. The number of viable *B. longum* cells in each culture was determined at intervals over 13 days. The results clearly demonstrate that growth *B*. *longum* strains in medium supplemented with AX-R or AX-W enhances strain survival during storage at 37°C as compared to cultures grown in medium supplemented with lactose (Fig. 2). For each *B. longum* strain tested there is a 6 log difference in culture viability following 13 days of storage of the cultures grown in medium supplemented with AX-R or AX-W as compared to *B. longum* cultures grown in medium supplemented with lactose (Fig. 2). During storage at 4°C we observe enhanced survival of *B. longum* strains NCIMB8809 and JCM1217 following growth in the medium supplemented with AX-R or AX-W as compared to lactose. For *B. longum* CCUG30698 we see equal survival of the three cultures over the 13 day time period suggesting that additional strain-specific factors may also play a role in the survival *B*. *longum* at 4°C. Collectively these results clearly demonstrate that survival of *B. longum* during storage at 37 °C or 4 °C is enhanced by growth in medium supplemented with AX-R or AX-W (Fig. 2).

*Isolation and enumeration of Bifidobacteria from fecal samples.* In order to establish if the inclusion of ferulic acid in growth medium would enhance the recovery of Bifidobacteria from infant fecal samples, TOS agar medium supplemented with 100 µg ml⁻¹ mupirocin and 50µg ml⁻¹ nystatin (TOS-MN) was prepared. Serial dilution of 2 infant faecal samples were prepared under anaerobic conditions and spread plated on TOS-MN with or without the inclusion of 2mM Ferulic acid. Following 48h incubation at 37 °C, under anaerobic conditions, bifidobacterial cell numbers were enumerated by plate counting. The results for each biological sample clearly demonstrate that the inclusion of ferulic acid in the agar medium results in at least a 0.5 log increase in recovery of bifidobacteria (Fig. 3).

*Recovery of Bifidobacteria following electrotransformation and site specific homologous recombination.* Site specific homologous recombination is a particularly low frequency event in Bifidobacteria and while electrocompetent cells may generate >1 × 10⁶ transformants µg⁻¹ plasmid DNA transformed, the recovery of insertion mutants from the same batch of electrocompetent cells can be < 10 colonies µg⁻¹ of transformed DNA. The inclusion of 1mM ferulic acid in the electrotransformation recovery medium, and in the agar plates has resulted in the recovery of bifidobacterial insertion mutants while in the absence of ferulic acid no mutants colonies were obtained.

## Claims

1. A method of growing an acid or oxygen sensitive strain of *Bifidobacterium* or *Lactobacillus,* comprising the step of incubating the acid or oxygen sensitive strain of *Bifidobacteria* or *Lactobacillus* in a fermentation medium comprising a phenolic acid-donating natural hemicellulose component as a fermentable energy source under growth favouring conditions and a for a period of time sufficient to increase the bacterial cell mass, and recovering the grown strain of *Bifidobacterium* or *Lactobacillus,* wherein the acid or oxygen sensitive strain of *Bifidobacterium* or *Lactobacillus* exhibits enhanced survival post-fermentation compared with the same bacteria grown in the fermentation medium without the phenolic acid-donating natural hemicellulose component.

2. A method as claimed in Claim 1 in which the phenolic acid-donating natural hemicellulose component is an arabinoxylan.

3. A method as claimed in Claim 1 or 2 in which the phenolic acid donating natural hemicellulose component comprises at least 75% of the fermentable energy source in the fermentation medium.

4. A method according to any preceding Claim in which the *Bifidobacterium* or *Lactobacillus* are subsequently dried.

5. A method according to Claim 4 in which the dried *Bifidobacterium* or *Lactobacillus* are stored in a storage medium, wherein the storage medium comprises the phenolic acid donating natural hemicellulose component.

6. A method according to Claim 4 or 5 in which the dried *Bifidobacterium* or *Lactobacillus* are reconstituted in a reconstitution medium comprising the phenolic acid donating natural hemicellulose component.

7. A method as claimed in any preceding Claim in which the *Bifidobacterium* or *Lactobacillus* are incorporated into a comestible product which additionally comprises the phenolic acid-donating natural hemicellulose component.

8. A method as claimed in any preceding Claim in which the comestible product is a fermented food product.

9. A culture of *Bifidobacterium* or *Lactobacillus* produced according to a method of any of Claims 1 to 6.

10. A culture of *Bifidobacterium* or *Lactobacillus* in a dried format produced according to a method of Claim 4.

11. A culture of *Bifidobacterium* or *Lactobacillus* in a dried and reconstituted format produced according to a method of Claim 6.

12. A fermentation medium suitable for growing *Bifidobacterium* or *Lactobacillus* comprising a fermentable energy source, and one or more growth factors selected from free amino acid, nucleotides, vitamins, minerals, **characterised in that** the fermentable energy source comprises a phenolic acid-donating natural hemicellulose.

13. A fermentation medium as claimed in Claim 12, in which the phenolic acid donating natural hemicellulose is arabinoxylan.

14. A fermentation medium as claimed in Claim 12 or 13, in which the phenolic acid donating natural hemicellulose is the sole fermentable energy source in the fermentation medium.
